# EUROPEAN PATENT APPLICATION

(11) **EP 4 624 591 A1**
(43) Date of publication of application: **01.10.2025**
(21) Application number: 23894621.4
(22) Date of filing: 22.11.2023
(51) Int. Cl.: C12Q 1/686, C12Q 1/6876

(54) **INTERNAL STANDARD GENE IN SKIN SURFACE LIPID SPECIMEN**

(30) Priority: 24.11.2022 JP 2022187310
(71) Applicant: Kao Corporation, Chuo-ku, Tokyo 103-8210 (JP)
(72) Inventor: IWAMURA, Maeko, Haga-gun, Tochigi 321-3497 (JP); UEDA, Yui, Haga-gun, Tochigi 321-3497 (JP); KUWANO, Tetsuya, Haga-gun, Tochigi 321-3497 (JP); FUSE, Chie, Haga-gun, Tochigi 321-3497 (JP); SHIMA, Kyoko, Haga-gun, Tochigi 321-3497 (JP)
(74) Representative: Vossius & Partner Patentanwälte Rechtsanwälte mbB
(86) International application number: PCT/JP2023/041940
(87) International publication number: WO 2024/111617

(57) **Abstract**

Provided are an internal reference gene for use in measuring an expression level of a target gene contained in a skin surface lipid specimen of an infant or young child, and a method for measuring an expression level of a target gene contained in a skin surface lipid specimen of an infant or young child using the internal reference gene. The present invention provides a method for measuring an expression level of a target gene contained in a skin surface lipid specimen of an infant or young child, comprising using at least one gene selected from the group consisting of particular 30 genes as an internal reference gene in PCR.

## Description

### Field of the Invention

The present invention relates to an internal reference gene in a skin surface lipid specimen and a gene expression level measurement method using the same.

### Background of the Invention

In the expression analysis of target genes contained in specimens, PCR typified by real-time PCR, which is excellent in convenience and is capable of analyzing even a trace amount of DNA or RNA, has been commonly used in recent years, though Northern blot had been used before then. An absolute quantification technique and a relative quantification technique are broadly used for the quantitative analysis of expression levels of target genes by the real-time PCR. The absolute quantification technique is an approach of preparing a calibration curve from reference samples having a known absolute amount such as a copy number, and quantifying an absolute amount of an expression level of a target gene in a specimen using this calibration curve. By contrast, the relative quantification technique is an approach of correcting an expression level of a target gene in a specimen with an expression level of an internal reference gene, and quantifying a relative expression level of the target gene.

For the internal reference gene, it is important to be a gene which does not vary in expression level among tissues or experimental systems. In general, a housekeeping gene such as GAPDH or ACTB is used. However, it has been reported that even such housekeeping genes may vary in expression depending on tissues and be not capable of functioning as internal reference genes (Non Patent Literatures 1 and 2).

Housekeeping genes including GAPDH, 1B15, RPLPO, actin, and tubulin have been used as internal reference genes in studies using epidermal keratinocytes. Nonetheless, it has been reported that not all of the housekeeping genes are suitable as internal reference genes, though RPLPO is useful as an internal reference gene (Non Patent Literature 3).

Meanwhile, Patent Literature 1 states that skin surface lipids (SSLs) contain RNA derived from skin cells of a test subject; the RNA contained in the SSLs is useful as a sample for *in vivo* gene expression analysis; and marker genes for epidermis, sweat gland, hair follicle, and sebaceous gland can be detected from the SSLs. However, any proper internal reference gene for gene expression analysis using SSLs has not yet been elucidated.

(Patent Literature 1) WO 2018/008319
(Non Patent Literature 1) Journal of Investigative Dermatology, 2009, 129 (3): 535-537
(Non Patent Literature 2) Acta Biochem Biophys Sin, 2014, 46 (4): 330-337
(Non Patent Literature 3) Journal of Investigative Dermatology, 2009, 129 (3): 770-773

### Summary of the Invention

The present invention relates to the following (1) to (3).
(1) A method for measuring an expression level of a target gene contained in an infant or young child SSL specimen, comprising using at least one gene selected from the group consisting of genes shown in Table 1 below as an internal reference gene in PCR.
(2) A kit for measurement of an expression level of a target gene contained in an infant or young child SSL specimen used in the method according to (1), the kit comprising an oligonucleotide which specifically hybridizes with the internal reference gene.
(3) Use of at least one gene selected from the group consisting of genes shown in Table 1 below as an internal reference gene in measurement of an expression level of a target gene contained in an infant or young child SSL specimen using PCR.

### Detailed Description of the Invention

All patent literatures, non patent literatures, and other publications cited herein are incorporated herein by reference in their entirety.

In the present specification, the term "nucleic acid", "nucleotide", "oligonucleotide", or "polynucleotide" means DNA or RNA. The DNA includes all of cDNA, genomic DNA, and synthetic DNA. The RNA includes all of total RNA, mRNA, rRNA, tRNA, non-coding RNA, and synthetic RNA.

In the present specification, the term "gene" encompasses double-stranded DNA including human genomic DNA as well as single-stranded DNA (positive strand) including cDNA, single-stranded DNA having a sequence complementary to the positive strand (complementary strand), and their fragments, and means a material containing some biological information in sequence information on bases constituting DNA.

The "gene" encompasses not only a "gene" represented by a particular nucleotide sequence but nucleic acids encoding its congeners (i.e., homologs or orthologs), variants such as gene polymorphisms, and derivatives.

The names (Gene Symbols) and Gene IDs disclosed in the present specification abide by Official Symbols and Gene IDs described in NCBI ([www.ncbi.nlm.nih.gov/]).

In the present specification, the term "internal reference gene", also called endogenous control gene or reference gene, means a gene which serves as a standard for the correction (standardization) of an expression level of a target gene in gene expression analysis. The "correction" is typically made by dividing an expression level of the target gene by an expression level of the internal reference gene. The term "expression level" or "amount" encompasses an absolute expression level and a relative expression level to an expression level of another gene or the like.

In the present specification, the term "housekeeping gene" is a gene indispensable for the maintenance or proliferation of cells and means a gene which is expressed in a certain amount in common among many tissues or cells. For example, GAPDH and ACTB are known as general housekeeping genes (see Genome Analysis, 2003, 19 (7): 362-365).

In the present specification, the term "infant or young child" conceptually includes a "child" before the onset of secondary sex characteristics, specifically a child at age 15 years or younger, in the broad sense, and preferably refers to a preschool child or an infant or young child, specifically, a 0- to 6-year-old infant or young child, more preferably 0- to 69-month-old infant or young child.

In the present specification, the term "skin surface lipids (SSLs)" refers to lipid-soluble fractions which reside on the surface of the skin, and is also called sebum. In general, SSLs mainly contain secretions from exocrine such as sebaceous gland in the skin, and reside on the skin surface in the form of a thin layer covering the skin surface. SSLs contain RNA expressed in skin cells (see Patent Literature 1 above). In the present specification, SSLs collected from a test infant or young child subject are also referred to as an SSL specimen.

In the present specification, the term "skin" is a generic name for regions containing tissues such as stratum corneum, epidermis, dermis, hair follicle, and sweat gland, sebaceous gland, and other glands, unless otherwise specified.

The present invention relates to provision of an internal reference gene for more accurately measuring an expression level of a target gene contained in infant or young child SSLs, and a method for measuring an expression level of a target gene contained in infant or young child SSLs using the internal reference gene.

The present inventors analyzed exhaustive gene expression data on infant or young child SSL specimens, thereby finding 30 genes having small differential expression among the specimens, and finding that these genes are excellent as internal reference genes in quantitative PCR (qPCR).

The internal reference gene of the present invention has small differential expression among infant or young child SSL specimens, particularly, small differential expression as compared with GAPDH and ACTB, which are known housekeeping genes generally used as internal reference genes. Use of the internal reference gene of the present invention as an internal reference gene in qPCR enables an expression level of a target gene contained in infant or young child SSLs to be more accurately measured.

The 30 genes shown in Table 1 of the present invention are genes found to have small variation in expression level of SSL-derived RNA among test infant or young child subjects, as shown in Examples mentioned later.

**[Table 1]**

| Gene Symbol | Gene ID | Gene Symbol | Gene ID |
|---|---|---|---|
| PCBP2 | 5094 | ARF1 | 375 |
| RPL30 | 6156 | RPS10 | 6204 |
| ARPC2 | 10109 | RPL11 | 6135 |
| UBA52 | 7311 | RPS19 | 6223 |
| RAB7A | 7879 | FTL | 2512 |
| RAC1 | 5879 | RPL12 | 6136 |
| ARL8B | 55207 | CLIC1 | 1192 |
| RPL32 | 6161 | CFL1 | 1072 |
| PCBP1 | 5093 | ARPC4 | 10093 |
| BZW1 | 9689 | RAB11FIP1 | 80223 |
| H3F3A | 3020 | NDUFA1 | 4694 |
| RPS8 | 6202 | PSMB4 | 5692 |
| FAU | 2197 | PSAP | 5660 |
| RPL4 | 6124 | GNB2L1 | 10399 |
| EEF1G | 1937 | CD63 | 967 |

The 30 genes shown in Table 1 are genes which exhibited stable expression as a result of using RLE as a normalization approach to normalize expression level data by using genes with a gene detection rate of 70% or more as analytes on the basis of data (read count values) on extracted RNA expression levels from SSLs of 261 test subjects of male and female infants or young children. The evaluation of expression stability employed a CV Z-score as an index. The "CV Z-score" indicates a deviation of a measurement value from a mean of a distribution and is a value (Z-score) which is obtained by dividing a difference between the measurement value and the mean of the distribution by a standard deviation of the distribution to calculate a coefficient of variation (CV), and standardizing thus calculated coefficient. A smaller value of the CV Z-score indicates smaller variation in gene expression (a mean of the CV Z-score itself is 0, and a standard deviation thereof is 1). In the present invention, a CV Z-score was used as an evaluation index for expression stability, and a gene having this score of -1.874 or lower was determined as a stably expressed gene. The 30 genes had an equivalent or smaller CV Z-score as compared with known housekeeping genes GAPDH (CV Z-score: -1.813) and ACTB (CV Z-score: - 1.874) reported to be generally suitable as internal reference genes, and RPLPO (CV Z-score: -1.859) suitable as an internal reference gene in the gene expression analysis of epidermal keratinocytes, as shown in Examples described later, and were thus confirmed to be stably expressed in SSLs.

Thus, a gene selected from the group consisting of these 30 genes can be used as an internal reference gene in qPCR for measuring an expression level of a target gene contained in infant or young child SSLs (hereinafter, also referred to as the internal reference gene of the present invention). Any one of the genes may be used singly as an internal reference gene, or two or more of the genes may be used in combination.

The internal reference gene of the present invention is preferably at least one gene selected from the group consisting of 16 genes PCBP2, RPL30, ARPC2, UBA52, RAB7A, RAC1, ARL8B, RPL32, PCBP1, BZW1, H3F3A, RPS8, FAU, RPL4, EEF1G, and ARF1, more preferably at least one gene selected from ARF1, RPL30, and FAU, further more preferably ARF1 or RPL30, further more preferably ARF1.

Among the 30 genes, 16 genes PCBP2, RPL30, UBA52, RAB7A, ARL8B, PCBP1, BZW1, RPS8, RPL4, EEF1G, FTL, RPL12, CLIC1, RAB11FIP1, PSAP, and CD63 are genes which have not been reported so far as housekeeping genes, and their capability of functioning as internal reference genes is totally unexpected. Accordingly, in one embodiment, the internal reference gene of the present invention is preferably at least one gene selected from the group consisting of the 16 genes, more preferably at least one gene selected from the group consisting of 10 genes PCBP2, RPL30, UBA52, RAB7A, ARL8B, PCBP1, BZW1, RPS8, RPL4, and EEF1G, further more preferably RPL30.

The internal reference gene of the present invention also encompasses a gene having a nucleotide sequence substantially identical to the nucleotide sequence of DNA constituting the gene as long as the gene is capable of serving as an internal reference in gene expression analysis. In this context, the substantially identical nucleotide sequence means that the sequence has 90% or more, preferably 95% or more, more preferably 98% or more identity to the nucleotide sequence of DNA constituting the gene in the case of search using, for example, homology calculation algorithm NCBI BLAST under conditions of expected value = 10; gap accepted; filtering = ON; match score = **1;** and mismatch score = -3.

The internal reference gene of the present invention is suitably used in a method for measuring an expression level of a target gene contained in an infant or young child SSL specimen (hereinafter, also referred to as the method of the present invention). In one embodiment, the method of the present invention includes, as to the infant or young child SSL specimen, correcting an amount of an amplification product of the target gene with an amount of an amplification product of the internal reference gene to calculate the expression level of the target gene. In a preferred embodiment, the method of the present invention includes, as to the infant or young child SSL specimen: amplifying the target gene; amplifying the internal reference gene; and correcting an amount of an amplification product of the target gene with an amount of an amplification product of the internal reference gene to calculate the expression level of the target gene. In a more preferred embodiment, the method of the present invention includes: extracting RNA from the SSL specimen collected from a test infant or young child subject; amplifying the target gene on the basis of the extracted RNA; amplifying the internal reference gene on the basis of the extracted RNA; and correcting an amount of an amplification product of the target gene with an amount of an amplification product of the internal reference gene to calculate the expression level of the target gene.

In the present invention, the target gene is not particularly limited, and can be a gene which may be contained in the infant or young child SSL specimen and may be one or more genes.

The collection of SSLs from the skin of the test infant or young child subject can adopt any approach for use in the recovery or removal of SSLs from the skin. Preferably, an SSL absorbent material, an SSL adhesive material, or a tool for scraping SSLs from the skin, as mentioned later can be used. The SSL absorbent material or the SSL adhesive material is not particularly limited as long as the material has affinity for SSLs. Examples thereof include polypropylene and pulp. More specific examples of the procedure for collecting SSLs from the skin include a method of allowing SSLs to be absorbed into a sheet-like material such as an oil-blotting paper and an oil-blotting film, and a method of allowing SSLs to be adhere to a glass plate, a tape, or the like, and a method of recovering SSLs by scraping the SSLs with a spatula, a scraper, or the like. An SSL absorbent material pre-impregnated with a highly lipid-soluble solvent may be used for improving the adsorbability of SSLs. On the other hand, an SSL absorbent material which contains a highly water-soluble solvent or water inhibits the adsorption of SSLs. Therefore, the SSL absorbent material preferably has a small content of the highly water-soluble solvent or water. The SSL absorbent material is preferably used in a dry state. Examples of the site of the skin from which SSLs are collected include, but not particularly limited to, the skin of an arbitrary site of the body, such as the head, the face, the neck, the trunk, and the limbs. A site having large secretion of sebum, for example, the skin of the face is preferred.

The RNA-containing SSL specimen collected from the test infant or young child subject may be immediately used in an RNA extraction step mentioned later or may be preserved for a given period. The collected SSL specimen is preferably preserved under a low-temperature condition as immediately after collection as possible for minimizing the degradation of the contained RNA. In the present invention, the preservation temperature condition of the RNA-containing SSLs can be 0°C or lower and is preferably from -20 ± 20°C to -80 ± 20°C, more preferably from -20 ± 10°C to -80 ± 10°C, further more preferably from -20 ± 20°C to -40 ± 20°C, further more preferably from -20 ± 10°C to -40 ± 10°C, further more preferably from -20 ± 10°C, further more preferably -20 ± 5°C. The preservation period under the low-temperature condition of the RNA-containing SSL specimen is not particularly limited and is preferably 12 months or shorter, for example, 6 hours or longer and 12 months or shorter, more preferably 6 months or shorter, for example, 1 day or longer and 6 months or shorter, further more preferably 3 months or shorter, for example, 3 days or longer and 3 months or shorter.

In a preferred aspect of the method of the present invention, RNA contained in the infant or young child SSL specimen is extracted and converted to cDNA by reverse transcription, and the target gene and the internal reference gene are amplified by PCR with the cDNA as a template, followed by the measurement of the amplification products.

The extraction of RNA from SSLs can employ a method which is usually used for extracting or purifying RNA from a biological sample, for example, a phenol/chloroform method, AGPC (acid guanidinium thiocyanate-phenol-chloroform extraction), a method using a column such as TRIzol(Registered Trademark), RNeasy(Registered Trademark), or QIAzol(Registered Trademark), a method using special magnetic particles coated with silica, a method using solid phase reversible immobilization magnetic particles, or extraction with a commercially available RNA extraction reagent such as ISOGEN.

The reverse transcription may employ primers targeting particular RNA to be analyzed, and preferably employs random primers for more comprehensive nucleic acid preservation and analysis. The reverse transcription can employ general reverse transcriptase or reverse transcription reagent kit. Reverse transcriptase or reverse transcription reagent kit having high accuracy and efficiency is suitably used. Examples thereof include M-MLV Reverse Transcriptase and its variants, and commercially available reverse transcriptase or reverse transcription reagent kits, for example, PrimeScript(Registered Trademark) Reverse Transcriptase series (Takara Bio Inc.) and SuperScript(Registered Trademark) Reverse Transcriptase series (Thermo Fisher Scientific Inc.). SuperScript (Registered Trademark) III Reverse Transcriptase, SuperScript (Registered Trademark) VILO cDNA Synthesis kit (both from Thermo Fisher Scientific Inc.), or the like is preferably used.

For extension reaction in the reverse transcription, it is preferred to adjust the temperature to preferably 42°C ± 1°C, more preferably 42°C ± 0.5°C, further more preferably 42°C ± 0.25°C, while it is preferred to adjust the reaction time to preferably 60 minutes or longer, more preferably from 80 to 120 minutes.

The amplification of the target gene or the internal reference gene with the cDNA obtained in reverse transcription as a template can be carried out in accordance with a procedure of PCR which is usually used in the art. Examples of the approach of PCR include conventional PCR, multiplex PCR, real-time PCR (also referred to as quantitative PCR (qPCR)), multiplex real-time PCR, and digital PCR.

In this context, cDNA synthesis by reverse transcription (RT) and PCR may be performed in one step or may be performed in two steps, and either one can be appropriately selected depending on a purpose. The onestep RT-PCR continuously performs a series of reactions of RT and PCR within a single tube, and is thus excellent in convenience and can prevent contamination between the operations of RT and PCR. On the other hand, the twostep RT-PCR can measure expression levels of a plurality of target genes from a single RNA specimen because random primers can be used in RT. Examples of the PCR in RT-PCR include the same as the PCR described above.

In PCR, only particular DNA to be analyzed may be amplified using a primer pair targeting the particular DNA, or a plurality of DNAs may be amplified at the same time using a plurality of primer pairs. Examples of the approach of amplifying a plurality of DNAs at the same time include multiplex PCR and multiplex real-time PCR. It is preferred to perform multiplex analysis in which the target gene and the internal reference gene are amplified at the same time, because expression levels can be more accurately measured by suppressing an error of an amount of template DNA. Such multiplex analysis can be carried out using a commercially available kit (e.g., Ion AmpliSeqTranscriptome Human Gene Expression Kit; Life Technologies Japan Ltd.).

A known approach which can specifically recognize an amplification product can be used in the detection of the amplification product. For example, a method of performing PCR using primers labeled in advance with RI, a fluorescent material, or the like, and detecting the labeled double-stranded DNA thus produced can be used.

In the present invention, real-time PCR or multiplex real-time PCR which monitors and analyzes an amount of a PCR amplification product in real time is preferably used as an approach of quantitative PCR (qPCR) from the viewpoint of swiftness, convenience, and quantitativeness. Examples of the method for detecting the amplification product in real-time PCR or multiplex real-time PCR include methods which are usually used in the art, for example, an intercalator method and a TaqMan(Registered Trademark) probe method.

The intercalator method is a method of allowing a substance which emits fluorescence through intercalation into double-stranded DNA (intercalator, for example, SYBR(Registered Trademark) Green I) to coexist in a PCR reaction system, and detecting fluorescence increased in association with the formation of an amplification product, thereby monitoring an amount of the amplification product.

The TaqMan probe method is a method of allowing a target sequence-specific oligonucleotide (TaqMan probe) modified at its 5' end with a fluorescent material (e.g., FAM) and at its 3' end with a quencher material (e.g., TAMRA) to coexist in a PCR reaction system. In this method, the TaqMan probe specifically hybridizes with template DNA in an annealing step of PCR reaction. In this state, the generation of fluorescence is suppressed due to the presence of the quencher material on the probe even when the probe is irradiated with excited light. Subsequently, the TaqMan probe hybridized with the template is degraded by the 5'→3' exonuclease activity of Taq DNA polymerase in an extension reaction step so that the fluorescent material is liberated from the probe to cancel the suppression by the quencher material, thereby emitting fluorescence. An amount of an amplification product can be monitored by detecting the fluorescence.

The PCR conditions are not particularly limited, and optimum conditions can be determined for each PCR. Examples thereof include the following conditions.
1) Thermal denaturation of double-stranded DNA into single-stranded DNA: The temperature is preferably from 94 to 99°C, and the time is from 10 to 60 seconds.
2) Annealing: The temperature is usually 50°C or higher, preferably 52°C or higher, more preferably 55°C or higher, and usually 65°C or lower, preferably 63°C or lower, more preferably 60°C or lower. The temperature is usually from 50 to 65°C, preferably from 52 to 63°C, more preferably from 55 to 60°C. The time is usually 5 seconds or longer, preferably 10 seconds or longer, and usually 2 minutes or shorter, preferably 1 minute or shorter. The time is usually from 5 seconds to 2 minutes, preferably from 10 seconds to 1 minute.
3) DNA extension reaction: The temperature is usually 65°C or higher, preferably 68°C or higher, and usually 74°C or lower, preferably 72°C or lower. The temperature is usually from 65 to 74°C, preferably from 68 to 72°C. The time is usually 5 seconds or longer, preferably 10 seconds or longer, and usually 2 minutes or shorter, preferably 1 minute or shorter. The time is usually from 5 seconds to 2 minutes, preferably from 10 seconds to 1 minute.

In this context, the annealing and the DNA extension reaction may be performed at the same time without being separated.

One cycle involves the reactions 1) to 3), and this operation can be performed at usually 30 or more cycles, preferably 35 or more cycles, and usually 50 or less cycles, preferably 45 or less cycles.

Reverse transcription and PCR using the temperatures and the times as described above can be performed using an apparatus dedicated to real-time PCR integrated with a thermal cycler or a thermal cycler and a spectrofluorometer which is generally used in PCR.

The strand length of an amplification product of PCR can be appropriately selected in consideration of a factor such as shortening of an amplification time of PCR. The strand length of the PCR amplification product is, for example, preferably 1 000 bp or less, more preferably 700 bp or less, further more preferably 500 bp or less. On the other hand, the lower limit of the strand length of the PCR amplification product is from 30 to 40 bp which is a strand length of a PCR amplification product in the case of using primers of approximately 15 bases which avoid nonspecific hybridization in PCR. The strand length is preferably 50 bp or more, more preferably 100 bp or more. The strand length of the PCR amplification product is preferably from 30 to 1 000 bp, more preferably from 50 to 700 bp, further more preferably from 100 to 500 bp.

Primers for specifically recognizing and amplifying the target gene and the internal reference gene, or nucleic acids derived therefrom, or probes for specifically detecting the target gene and the internal reference gene, or nucleic acids derived therefrom correspond to probes or primers for use in the measurement described above. These primers or probes can be designed on the basis of a nucleotide sequence constituting the target gene or the internal reference gene. In this context, the term "specifically recognize" means that, for example, in RT-PCR, a detected substance or a product can be determined as the target gene or the internal reference gene, or a nucleic acid derived therefrom as if only the gene or the nucleic acid derived therefrom is substantially amplified.

Specifically, an oligonucleotide can be used which contains a given number of nucleotides complementary to DNA consisting of a nucleotide sequence constituting the target gene or the internal reference gene of the present invention, or a given number of nucleotides complementary to a complementary strand of the DNA. In this context, the "complementary strand" refers to one strand for another strand of double-stranded DNA composed of A:T (or U for RNA) and G:C pairs. The term "complementary" is not limited to the case where a sequence is completely complementary in a region with the given number of consecutive nucleotides, and can have preferably 80% or more, more preferably 90% or more, further more preferably 95% or more, further more preferably 98% or more identity on a nucleotide sequence. The nucleotide sequence identity can be determined with an algorithm such as BLAST described above.

In the case of using such oligonucleotides as primers, the oligonucleotides may achieve specific annealing and strand extension. Examples thereof include primers having a strand length of usually 10 or more bases, preferably 15 or more bases, more preferably 20 or more bases, and 100 or less bases, preferably 50 or less bases, more preferably 35 or less bases. In the case of using such an oligonucleotide as a probe, the oligonucleotide may achieve specific hybridization. The probe used has at least a partial sequence or the whole sequence of DNA consisting of a nucleotide sequence constituting the target gene or the internal reference gene of the present invention (or a complementary strand of the DNA), and has a strand length of, for example, 10 or more bases, preferably 15 or more bases, and, for example, 100 or less bases, preferably 50 or less bases, more preferably 25 or less bases.

In this context, the "oligonucleotide" can be DNA or RNA and may be a synthetic or natural product. The probe for use in hybridization is usually labeled.

If the amount of template DNA in PCR is a trace amount or if many sequences are similar to a target sequence, the yield and specificity of an amplification product can be enhanced by nested PCR. In the nested PCR, a first round of PCR is performed using a first primer pair to amplify a target sequence. Subsequently, a second round of PCR can be performed with the amplification product as a template using a second primer pair designed for an internal area of the region amplified with the first primer pair to obtain an amplification product. Among these two rounds of PCR, at least the second round of PCR is preferably real-time PCR or multiplex real-time PCR from the viewpoint of quantitativeness.

In this way, the target gene and the internal reference gene are amplified on the basis of the RNA extracted from the infant or young child SSL specimen. The expression level of the target gene is calculated on the basis of the amplification products. The expression level of the target gene can be calculated in accordance with a relative quantification technique which is usually used in the art. Examples of the relative quantification technique include an approach of subjecting the amplification product of the target gene and the amplification product of the internal reference gene to electrophoresis, and comparing band densities on a gel to calculate a relative expression level of the target gene to the internal reference gene, a known calibration curve technique as a relative quantification approach of real-time PCR, a -ΔCt technique, and a ΔΔCt technique (comparative Ct technique). Among these relative quantification techniques, a -ΔCt technique or a ΔΔCt technique is preferred because no calibration curve is necessary.

In the case of using a calibration curve technique as a relative quantification approach, relative quantification can be performed, for example, as described below. First, a dilution series of reference samples is prepared, and each of the target gene and the internal reference gene is amplified by PCR with this dilution series as templates to determine Ct values. In this context, the Ct values mean cycle numbers when the amount of a PCR amplification product reaches a given amount (threshold cycle). In PCR, an amplification product theoretically increases twofold per cycle. Therefore, the concentrations of the reference samples and the determined Ct values, when plotted, are in a relationship represented by a straight line, and this plot can be used as a calibration curve. Subsequently, PCR is also performed under the same conditions as above as to the specimen to determine a Ct value. Results of quantifying the target gene and the internal reference gene in the specimen are obtained from this value and the calibration curve. Further, the results of quantifying the target gene are divided by the results of quantifying the internal reference gene so that the expression level of the target gene is corrected with the expression level of the internal reference gene to calculate the expression level of the target gene. The expression level of the target gene may be indicated by a relative amount when results about a certain specimen are defined as 1 so as to easily understand a difference in expression level between specimens.

In the case of using a -ΔCt technique as a relative quantification approach, relative quantification can be performed, for example, as described below. First, each of the target gene and the internal reference gene in specimen A is amplified by PCR to determine Ct values. A difference (ΔCt) between the Ct value of the target gene and the Ct value of the internal reference gene is calculated. In this context, the ΔCt is a value obtained by correcting results of quantifying the target gene with results of quantifying the internal reference gene. PCR is also performed under the same conditions as above as to specimen B to be compared to determine Ct values and ΔCt. Subsequently, the obtained ΔCt is substituted into an expression (2^{-ΔCt}) , and the expression level is compared between the specimens A and B on the basis of the calculated relative expression level.

In the case of using a ΔΔCt technique as a relative quantification approach, relative quantification can be performed, for example, as described below. First, each of the target gene and the internal reference gene in specimen A is amplified by PCR to determine Ct values. A difference (ΔCt) between the Ct value of the target gene and the Ct value of the internal reference gene is calculated. In this context, the ΔCt is a value obtained by correcting results of quantifying the target gene with results of quantifying the internal reference gene. PCR is also performed under the same conditions as above as to specimen B to be compared to determine Ct values and ΔCt. Subsequently, a difference between the ΔCt of the specimen A and the ΔCt of the specimen B (ΔΔCt) is determined. In this context, the ΔΔCt is a value which reflects a difference in expression level of the target gene between specimens. Further, the obtained ΔΔCt is substituted into an expression (2^{-ΔΔCt}). The value obtained here shows how many times the expression level of the target gene in the specimen A is higher or lower than the expression level of the target gene in the specimen **B.** Specifically, the expression level of the target gene in the specimen A is indicated by a relative amount with the expression level in the specimen B defined as **1.**

A kit for measurement of an expression level of a target gene contained in an infant or young child SSL specimen used in the method of the present invention, contains an examination reagent for measuring an expression level of the internal reference gene of the present invention in SSLs collected from a test infant or young child subject. Specific examples thereof include reagents for nucleic acid amplification or hybridization, containing an oligonucleotide (e.g., primers or a probe for PCR) which specifically binds (hybridizes) to the internal reference gene of the present invention or a nucleic acid derived therefrom. The oligonucleotide to be contained in the kit can be obtained by a known method as mentioned above.

The kit can also contain, in addition to the nucleic acid described above, reference samples for calibration curve preparations, an RT reagent, a PCR reagent, a labeling reagent, a buffer solution, an instrument or a control necessary for tests, a tool for collecting SSLs (e.g., an oil-blotting film for collecting SSLs), a reagent for preserving the collected SSLs, a container for preservation, and a reagent for extracting or purifying RNA from the collected SSLs, and the like.

In relation to the embodiments mentioned above, the present invention further discloses the following aspects.
<1> A method for measuring an expression level of a target gene contained in a skin surface lipid specimen of an infant or young child, comprising using at least one gene selected from the group consisting of genes shown in the following Table 2 as an internal reference gene in PCR.

**[Table 2]**

| Gene Symbol | Gene Symbol |
|---|---|
| PCBP2 | ARF1 |
| RPL30 | RPS10 |
| ARPC2 | RPL11 |
| UBA52 | RPS19 |
| RAB7A | FTL |
| RAC1 | RPL12 |
| ARL8B | CLIC1 |
| RPL32 | CFL1 |
| PCBP1 | ARPC4 |
| BZW1 | RAB11FIP1 |
| H3F3A | NDUFA1 |
| RPS8 | PSMB4 |
| FAU | PSAP |
| RPL4 | GNB2L1 |
| EEF1G | CD63 |

<2> The method according to <1>, further comprising correcting an amount of an amplification product of the target gene with an amount of an amplification product of the internal reference gene to calculate the expression level of the target gene.
<3> The method according to <1>, further comprising:
amplifying the target gene;
amplifying the internal reference gene; and
correcting an amount of an amplification product of the target gene with an amount of an amplification product of the internal reference gene to calculate the expression level of the target gene.

<4> The method according to <1>, further comprising:
extracting RNA from the skin surface lipid specimen collected from a test infant or young child subject;
amplifying the target gene on the basis of the extracted RNA;
amplifying the internal reference gene on the basis of the extracted RNA; and
correcting an amount of an amplification product of the target gene with an amount of an amplification product of the internal reference gene to calculate the expression level of the target gene.

<5> The method according to <1>, further comprising:
extracting RNA from the skin surface lipid specimen collected from a test infant or young child subject;
synthesizing cDNA with the extracted RNA as a template;
amplifying the target gene with the obtained cDNA as a template;
amplifying the internal reference gene with the obtained cDNA as a template; and
correcting an amount of an amplification product of the target gene with an amount of an amplification product of the internal reference gene to calculate the expression level of the target gene.

<6> The method according to any of <1> to <5>, wherein the internal reference gene is preferably at least one gene selected from the group consisting of PCBP2, RPL30, ARPC2, UBA52, RAB7A, RAC1, ARL8B, RPL32, PCBP1, BZW1, H3F3A, RPS8, FAU, RPL4, EEF1G, and ARF1, more preferably at least one gene selected from the group consisting of ARF1, RPL30, and FAU, further more preferably ARF1 or RPL30, further more preferably ARF1.
<7> The method according to any of <1> to <5>, wherein the internal reference gene is preferably at least one gene selected from the group consisting of PCBP2, RPL30, UBA52, RAB7A, ARL8B, PCBP1, BZW1, RPS8, RPL4, EEF1G, FTL, RPL12, CLIC1, RAB11FIP1, PSAP, and CD63, more preferably at least one gene selected from the group consisting of PCBP2, RPL30, UBA52, RAB7A, ARL8B, PCBP1, BZW1, RPS8, RPL4, and EEF1G, further more preferably RPL30.
<8> The method according to any of <1> to <7>, wherein the target gene and the internal reference gene are amplified by qPCR, preferably by multiplex qPCR.
<9> A kit for measurement of an expression level of a target gene contained in a skin surface lipid specimen of an infant or young child used in the method according to any of <1> to <8>, the kit comprising an oligonucleotide which specifically hybridizes with the internal reference gene.
<10> Use of at least one gene selected from the group consisting of genes shown in Table 2 above as an internal reference gene in measurement of an expression level of a target gene contained in a skin surface lipid specimen of an infant or young child using PCR.
<11> The use according to <10>, wherein the measurement of the expression level of the target gene further comprises correcting an amount of an amplification product of the target gene with an amount of an amplification product of the internal reference gene to calculate the expression level of the target gene.
<12> The use according to <10>, wherein the measurement of the expression level of the target gene further comprises:
amplifying the target gene;
amplifying the internal reference gene; and
correcting an amount of an amplification product of the target gene with an amount of an amplification product of the internal reference gene to calculate the expression level of the target gene.

<13> The use according to <10>, wherein the measurement of the expression level of the target gene further comprises:
extracting RNA from the skin surface lipid specimen collected from a test infant or young child subject;
amplifying the target gene on the basis of the extracted RNA;
amplifying the internal reference gene on the basis of the extracted RNA; and
correcting an amount of an amplification product of the target gene with an amount of an amplification product of the internal reference gene to calculate the expression level of the target gene.

<14> The use according to <10>, wherein the measurement of the expression level of the target gene further comprises:
extracting RNA from the skin surface lipid specimen collected from a test infant or young child subject;
synthesizing cDNA with the extracted RNA as a template;
amplifying the target gene with the obtained cDNA as a template;
amplifying the internal reference gene with the obtained cDNA as a template; and
correcting an amount of an amplification product of the target gene with an amount of an amplification product of the internal reference gene to calculate the expression level of the target gene.

<15> The use according to any of <10> to <14>, wherein the internal reference gene is preferably at least one gene selected from the group consisting of PCBP2, RPL30, ARPC2, UBA52, RAB7A, RAC1, ARL8B, RPL32, PCBP1, BZW1, H3F3A, RPS8, FAU, RPL4, EEF1G, and ARF1, more preferably at least one gene selected from the group consisting of ARF1, RPL30, and FAU, further more preferably at least one gene selected from the group consisting of ARF1 and RPL30, further more preferably ARF1.
<16> The use according to any of <10> to <14>, wherein the internal reference gene is preferably at least one gene selected from the group consisting of PCBP2, RPL30, UBA52, RAB7A, ARL8B, PCBP1, BZW1, RPS8, RPL4, EEF1G, FTL, RPL12, CLIC1, RAB11FIP1, PSAP, and CD63, more preferably at least one gene selected from the group consisting of PCBP2, RPL30, UBA52, RAB7A, ARL8B, PCBP1, BZW1, RPS8, RPL4, and EEF1G, further more preferably RPL30.
<17> The use according to any of <10> to <16>,
wherein the target gene and the internal reference gene are amplified by qPCR, preferably by multiplex qPCR.

### Examples

Hereinafter, the present invention will be described in more detail with reference to Examples. However, the present invention is not limited thereto.

### Example 1 Collection of SSLs, RNA extraction, and exhaustive gene expression analysis

### 1) Test subject

Test subjects consisted of 261 male and female infants or young children (0 to 69 months old).

### 2) Collection of SSLs

Skin surface lipids (SSLs) were collected from the whole face of each test subject using an oil-blotting film (5.0 cm × 8.0 cm, 3M Company). In order to prevent the cross contamination of sebum, laboratory gloves of a collector were replaced with new ones on a test subject basis. The oil-blotting film with the collected sebum was immediately placed in a RNase-free centrifugal tube (AGC Techno Glass Co., Ltd., Centrifugal Tube Mini 25 mL) containing 1 g of molecular sieves, or a 5 mL tube (Eppendorf DNA LoBind 5 mL, PCR clean) containing 1 g of molecular sieves, and this container was left standing on dry ice or at -20°C and then stored at -80°C.

### 3) RNA extraction and sequencing

The oil-blotting film with the collected sebum in 2) above was cut into an appropriate size, and RNA was extracted using QIAzol Lysis Reagent (Qiagen N.V.) in accordance with the attached protocol. On the basis of the extracted RNA, cDNA was synthesized by reverse transcription at 42°C for 90 minutes using SuperScript VILO cDNA Synthesis kit (Life Technologies Japan Ltd.). Random primers attached to the kit were used as primers for the reverse transcription reaction.

A library containing DNAs derived from 20 802 genes was prepared by multiplex PCR from the obtained cDNA. The multiplex PCR was performed using Ion AmpliSeqTranscriptome Human Gene Expression Kit (Life Technologies Japan Ltd.) under conditions of [99°C for 2 minutes → (99°C for 15 seconds → 62°C for 16 minutes) × 20 cycles → 4°C, hold]. The obtained PCR products were purified with Ampure XP (Beckman Coulter Inc.), followed by buffer reconstitution, primer sequence digestion, adapter ligation, purification, and amplification to prepare a library.

The prepared library was loaded in Ion 540 Chip and sequenced using Ion S5/XL system (Life Technologies Japan Ltd.). Each read sequence obtained by sequencing was genetically mapped to a reference sequence of the human genome, hg19 AmpliSeq Transcriptome ERCC v1, to determine a gene from which each read sequence was derived.

### Example 2 Identification of internal reference gene

On the basis of the data obtained in Example 1, the following analysis was performed.

Genes with a gene detection rate of 70% or more were normalized as analytes (gene threshold) by RLE. RLE is an approach of calculating a coefficient called size factor, and normalizing read count data on each sample through the use of this coefficient. Genes were selected which exhibited a constant rate of stable expression in the correction data thus normalized. Gene expression stability was evaluated through the use of CV Z-scores. As a result of extracting genes having higher expression stability with a CV Z-score of -1 or lower in the correction data, 1636 genes were extracted. These genes which exhibited CV Z-score ≤ -1 corresponded to top 18.88% analyte genes having small differential expression. GAPDH and ACTB which are generally used as internal reference genes have a CV Z-score of GAPDH = - 1.813 and ACTB = -1.874. RPLPO reportedly suitable as an internal reference gene in the gene expression analysis of epidermal keratinocytes had a CV Z-score of -1.859.

Accordingly, among the genes having high expression stability with CV Z-score ≤ -1, genes which had an equivalent or smaller CV Z-score as compared with ACTB and were more stably expressed were further selected to identify a total of 30 genes (Table 3). In the table, reported housekeeping genes were marked with * (see Genome Analysis, 2003, 19 (7): 362-365).

The 30 genes were genes which were more stably expressed in infant or young child SSL-derived RNA, and were found usable as internal reference genes in the case of using infant or young child SSLs as samples to be analyzed, and analyzing their gene expression.

**[Table 3]**

| Gene Symbol\| | CV_Z-score | | Gene Symbol\| | CV_Z-score | |
|---|---|---|---|---|---|
| PCBP2 | -1.971 | | ARF1 | -1.900 | * |
| RPL30 | -1.960 | | RPS10 | -1.899 | * |
| ARPC2 | -1.956 | * | RPL11 | -1.898 | * |
| UBA52 | -1.953 | | RPS19 | -1.896 | * |
| RAB7A | -1.952 | | FTL | -1.895 | |
| RAC1 | -1.945 | * | RPL12 | -1.894 | |
| ARL8B | -1.934 | | CLIC1 | -1.894 | |
| RPL32 | -1.923 | * | CFL1 | -1.892 | * |
| PCBP1 | -1.920 | | ARPC4 | -1.888 | * |
| BZW1 | -1.913 | | RAB11FIP1 | -1.887 | |
| H3F3A | -1.911 | * | NDUFA1 | -1.886 | * |
| RPS8 | -1.910 | | PSMB4 | -1.880 | * |
| FAU | -1.905 | * | PSAP | -1.878 | |
| RPL4 | -1.903 | | GNB2L1 | -1.876 | * |
| EEF1G | -1.901 | | CD63 | -1.874 | |

### Example 3 Confirmation of correlation between real-time PCR data and NGS data - 1

Sebum was collected from each of 15 infants or young children (3 to 8 months old), and RNA extraction and purification were performed. The SSL RNA and RT Mix were mixed, and reverse transcription was performed. A library containing DNAs derived from 20 802 genes was prepared by multiplex PCR from the obtained reverse transcription reaction products (1st round PCR, Ion AmpliSeqTranscriptome Human Gene Expression Kit). The obtained 1st round PCR products were purified using Ampure XP. The obtained purification products were divided into one for next-generation sequencer analyses and one for real-time PCR measurements. The one purification product was subjected to buffer reconstitution, primer sequence digestion, adapter ligation, purification, and amplification, and exhaustive gene expression information was obtained by next-generation sequencer analysis and used as NGS data. The other 1st round PCR purification product was used as real-time PCR measurement templates and primers designed for an internal area of the 1st round PCR-amplified region (nested primers) were used to perform real-time PCR. Ct values of 14 genes selected as target genes and ARF1 as internal reference genes (control genes) were obtained as to each of the 15 samples. A difference Ct value (ΔCt value; ΔCt = Ct__{target gene} - Ct__{control gene}) was calculated and used as qPCR data. As a result of confirming data correlation between the qPCR data and the NGS data (Log₂(normalized count + 1)) as to the 14 target genes, significant positive correlation was obtained (Spearman's rank correlation coefficient = 0.780, p-value < 0.01), and the qPCR data calculated by ARF1 correction was thus confirmed to be equivalent with the NGS data.

### Example 4 Confirmation of correlation between real-time PCR data and NGS data - 2

Sebum was collected from each of 16 infants or young children (3 to 24 months old), and RNA extraction and purification were performed. The SSL RNA and RT Mix were mixed, and reverse transcription was performed. A library containing DNAs derived from 20 802 genes was prepared by multiplex PCR from the obtained reverse transcription reaction products (1st round PCR, Ion AmpliSeqTranscriptome Human Gene Expression Kit). The obtained 1st round PCR products were purified using Ampure XP. The obtained purification products were divided into one for next-generation sequencer analyses and one for real-time PCR measurements. The one purification product was subjected to buffer reconstitution, primer sequence digestion, adapter ligation, purification, and amplification, and exhaustive gene expression information was obtained by next-generation sequencer analysis and used as NGS data. The other 1st round PCR purification product was used as real-time PCR measurement templates and primers designed for an internal area of the 1st round PCR-amplified region (nested primers) were used to perform real-time PCR. Ct values of 14 genes selected as target genes and 6 genes (ARF1, FAU, RPL30, GAPDH, ACTB, and RPLPO) as internal reference genes (control genes) were obtained as to each of the 16 samples. A difference Ct value (ΔCt value: Ct__{target gene} - Ct_{_control gene}) was calculated for each of the 6 internal reference genes and used as qPCR data (hereinafter, referred to as ΔARF1, ΔFAU, ΔRPL30, ΔGAPDH, ΔACTB, and ΔRPLP0). Data correlation between the qPCR data and the NGS data (Log₂(normalized count + 1)) was confirmed as to the 14 target genes. It was confirmed, as shown in Table 4 below, that as compared with the qPCR data (ΔGAPDH, ΔACTB, and ΔRPLP0) obtained from internal reference GAPDH (CV Z-score = -1.813) and ACTB (CV Z-score = -1.874), which are generally used as internal reference genes, and PRLP0 (CV Z-score = -1.859) considered suitable as an internal reference gene in gene expression analysis on epidermal keratinocytes, qPCR data (ΔARF1, ΔFAU, and ΔRPL30) obtained from the internal reference ARF1 (CV Z-score = -1.900), FAU (CV Z-score = - 1.905), and RPL30 (CV Z-score = -1.960) selected as the SSL internal reference genes of the present invention improved the correlation of the whole PCR quantification data on the target genes, and are superior as internal reference genes for measuring expression levels of target genes contained in a skin surface lipid specimen of an infant or young child.

**[Table 4]**

| NGS data v.s. qPCR data | ΔARF1 | ΔFAU | ΔRPL30 | ΔGAPDH | ΔACTB | ΔRPLP0 |
|---|---|---|---|---|---|---|
| Spearman's rank correlation coefficient | 0.9244 | 0.9198 | 0.9452 | 0.9172 | 0.9197 | 0.9130 |

## Claims

1. A method for measuring an expression level of a target gene contained in a skin surface lipid specimen of an infant or young child, comprising using at least one gene selected from the group consisting of genes shown in the following Table 1 as an internal reference gene in PCR.
**[Table 1]**
| Gene Symbol | Gene Symbol |
|---|---|
| PCBP2 | ARF1 |
| RPL30 | RPS10 |
| ARPC2 | RPL11 |
| UBA52 | RPS19 |
| RAB7A | FTL |
| RAC1 | RPL12 |
| ARL8B | CLIC1 |
| RPL32 | CFL1 |
| PCBP1 | ARPC4 |
| BZW1 | RAB11FIP1 |
| H3F3A | NDUFA1 |
| RPS8 | PSMB4 |
| FAU | PSAP |
| RPL4 | GNB2L1 |
| EEF1G | CD63 |

2. The method according to claim 1, comprising correcting an amount of an amplification product of the target gene with an amount of an amplification product of the internal reference gene to calculate the expression level of the target gene.

3. The method according to claim 1 or 2,
wherein the internal reference gene is at least one gene selected from the group consisting of ARF1, RPL30, and FAU.

4. A kit for measurement of an expression level of a target gene contained in a skin surface lipid specimen of an infant or young child used in the method according to any one of claims 1 to 3, the kit comprising an oligonucleotide which specifically hybridizes with the internal reference gene.

5. Use of at least one gene selected from the group consisting of genes shown in Table 1 above as an internal reference gene in measurement of an expression level of a target gene contained in a skin surface lipid specimen of an infant or young child using PCR.

6. The use according to claim 5, wherein the measurement of the expression level of the target gene comprises correcting an amount of an amplification product of the target gene with an amount of an amplification product of the internal reference gene to calculate the expression level of the target gene.

7. The use according to claim 5 or 6, wherein the internal reference gene is at least one gene selected from the group consisting of ARF1, RPL30, and FAU.
